# EUROPEAN PATENT APPLICATION

(11) **EP 4 223 865 A1**
(43) Date of publication of application: **09.08.2023**
(21) Application number: 21875078.4
(22) Date of filing: 03.09.2021
(51) Int. Cl.: C12M 1/26, G01N 1/04

(54) **FINE PARTICLE CAPTURE DEVICE**

(30) Priority: 29.09.2020 JP 2020163376
(71) Applicant: NOK Corporation, Tokyo 105-8585 (JP)
(72) Inventor: KOMORI, Takayuki, Fujisawa-shi, Kanagawa 251-0042 (JP)
(74) Representative: Global IP Europe Patentanwaltskanzlei
(86) International application number: PCT/JP2021/032514
(87) International publication number: WO 2022/070776

(57) **Abstract**

The present invention addresses the problem of providing a fine particle capture device capable of capturing a single particle by simply causing a fine particle-containing liquid to flow through a flow channel without using electricity or performing a special operation. Said problem is solved by this fine particle capture device for capturing fine particles by causing a fine particle-containing liquid to pass through the device. In the fine particle capture device, a chip has a plane part and projection parts, and is configured such that the fine particle-containing liquid entering from an inlet port passes on the surface of the plane part in the chip and between a projection part and another projection part adjacent thereto and the liquid is discharged from an outlet port. The projection parts are disposed on the plane part in the form of layers. The layers each have a plurality of projection parts, and are configured such that the fine particle-containing liquid having passed through a layer on the inlet port side passes through a layer on the outlet port side adjacent thereto. In each layer, a capture part and a bypass part are formed. The capture part is disposed on the outlet port side of the bypass part in a specific layer as a portion of another layer adjacent thereto.

## Description

### TECHNICAL FIELD

The present invention relates to a fine particle capture device.

### BACKGROUND ART

At present, bulk analysis is used as a method of examining properties of living cells. The bulk analysis is a method which involves collecting/destroying a cell population to analyze genes or proteins. On the other hand, this method contains information of all collected cells and therefore information of non-target cells is also reflected in data. It was also found out in recent years that even if cells are of the same species, expression of their genes is different and it has been desired to establish an analysis method on a single-cell level. Cell capture on the single-cell level is required to realize this.

A method in which cells are captured in wells using a dielectrophoresis technique (see Patent Document 1 and Non-Patent Document 1) and a method in which cells are directly manipulated/captured (see Non-Patent Document 2) are proposed as methods of realizing single cell capture.

### CITATION LIST

### PATENT DOCUMENTS

Patent Document 1: JP 2012-34641 A

### NON-PATENT DOCUMENTS

Non-Patent Document 1: Atsushi MORIMOTO et al., Detection and Single Cell Genomic Analysis of Circulating Tumor Cells from Metastatic Breast Cancer Patients, TOSOH Research & Technology Review, Vol.59 (2015)
Non-Patent Document 2: Masato SAITO et al., Development of the Micro PCR Devices for Single Cell DNA Analysis, Transactions of Japanese Society for Medical and Biological Engineering, 51(3) : 211-216,2013

### SUMMARY OF THE INVENTION

### PROBLEM TO BE SOLVED BY THE INVENTION

In these methods, however, the devices require special structures and skilled manipulation techniques are necessary, and further screening depending on the species of cells is also difficult.

In addition to the aforementioned methods of examining properties of living cells, a technique of singly separating fine particles has been sought to analyze fine particulate matter such as PM10 and PM2.5, and microplastic particles.

An object of the present invention is to solve the problem as described above. Specifically, an object of the present invention is to provide a fine particle capture device capable of capturing single particles by merely flowing a fine particle-containing liquid in flow paths without electricity or any special manipulation.

### MEANS FOR SOLVING THE PROBLEM

The present invention provides the following (1) and (2).
(1) A fine particle capture device including: a chip for passing a fine particle-containing liquid therethrough and capturing fine particles contained in the fine particle-containing liquid,
   wherein the chip has a flat part and a large number of protruding parts provided thereon, and is configured so that the fine particle-containing liquid having entered through an inlet passes on a surface of the flat part in the chip, and through spaces each located between a protruding part and another protruding part adjacent thereto and is discharged from an outlet,
   wherein the protruding parts are provided on the flat part in a layered form, each layer has a plurality of protruding parts, and the protruding parts are configured so that the fine particle-containing liquid having passed through a layer on an inlet side passes through a layer adjacent thereto on an outlet side,
   wherein capture parts and bypass parts are formed in each layer, a width between a protruding part and its adjacent protruding part being set to be smaller in the capture parts and larger in the bypass parts than a diameter of the fine particles to be captured, and
   wherein capture parts are disposed on an outlet side of bypass parts in a specific layer as a part of another layer adjacent thereto.
(2) The fine particle capture device according to (1) above, wherein the protruding parts have a rectangular or approximately rectangular shape when seen from a direction perpendicular to a main surface of the chip.

### EFFECT OF THE INVENTION

The present invention can provide a fine particle capture device capable of capturing single particles by merely flowing a fine particle-containing liquid in flow paths without electricity or any special manipulation.

### BRIEF DESCRIPTION OF THE DRAWINGS

[FIG. 1] FIG. 1 is a schematic view of a chip surface of a fine particle capture device in a preferred embodiment of the invention.
[FIG. 2] FIG. 2 is a cross-sectional view taken along line B-B in FIG. 1.
[FIG. 3] FIG. 3 is an enlarged view of a portion A in FIG. 1.
[FIG. 4] FIG. 4 is an enlarged photo of a surface of a chip used in Examples.
[FIG. 5] FIG. 5 is an enlarged photo of a chip obtained by observing with a stereoscopic microscope, the photo showing the state of fine particles captured in Examples.

### DESCRIPTION OF EMBODIMENTS

The fine particle capture device of the invention is a fine particle capture device including: a chip for passing a fine particle-containing liquid therethrough and capturing fine particles contained in the fine particle-containing liquid, wherein the chip has a flat part and a large number of protruding parts provided thereon, and is configured so that the fine particle-containing liquid having entered through an inlet passes on a surface of the flat part in the chip, and through spaces each located between a protruding part and another protruding part adjacent thereto and is discharged from an outlet, wherein the protruding parts are provided on the flat part in a layered form, each layer has a plurality of protruding parts, and the protruding parts are configured so that the fine particle-containing liquid having passed through a layer on an inlet side passes through a layer adjacent thereto on an outlet side, wherein capture parts and bypass parts are formed in each layer, a width between a protruding part and its adjacent protruding part being set to be smaller in the capture parts and larger in the bypass parts than a diameter of the fine particles to be captured, and wherein capture parts are disposed on an outlet side of bypass parts in a specific layer as a part of another layer adjacent thereto.

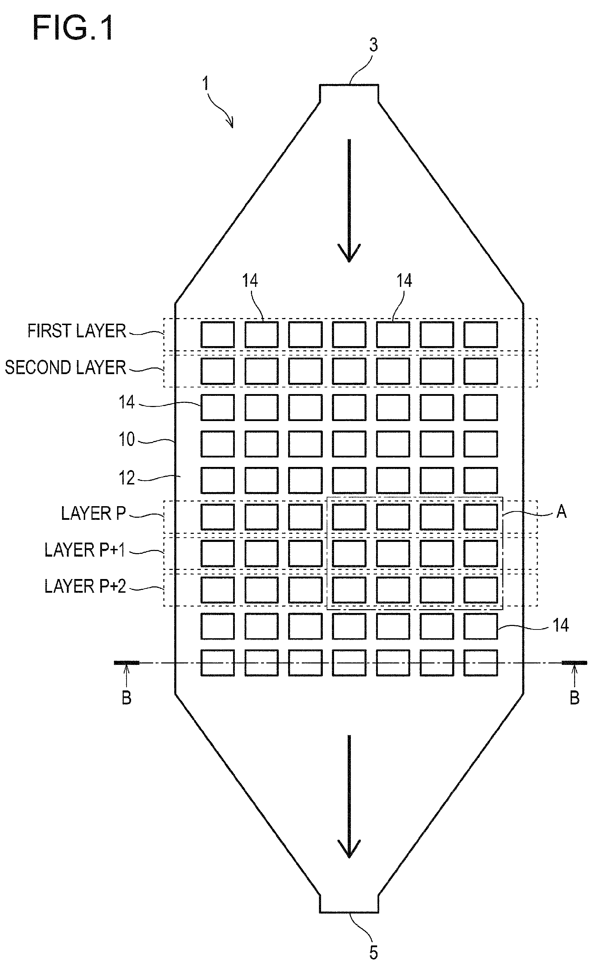

The fine particle capture device of the invention is described with reference to FIG. 1 to FIG. 3.

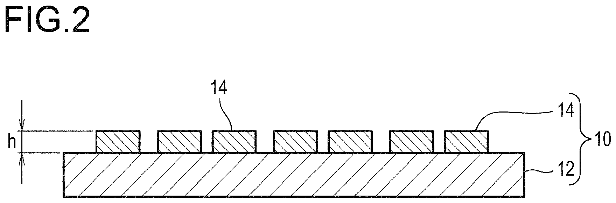

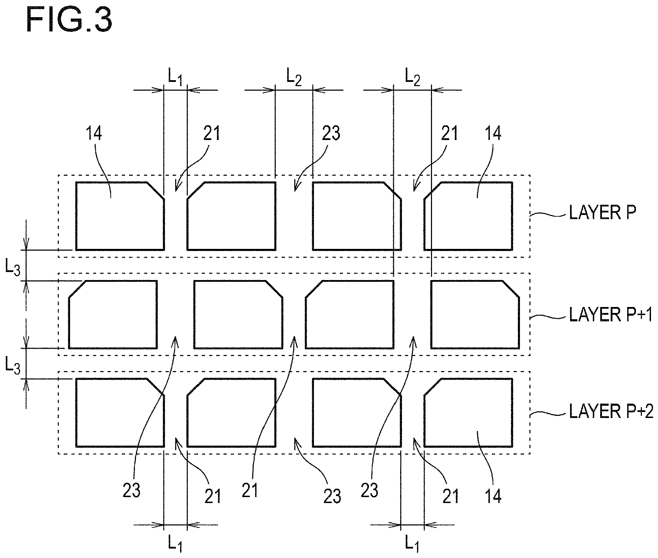

FIG. 1 is a schematic view showing a main surface of a chip 10 in a fine particle capture device 1 of the invention. FIG. 2 is a cross-sectional view taken along line B-B in FIG. 1. FIG. 3 is an enlarged view of a portion A in FIG. 1.

The fine particle device 1 of the invention illustrated in FIG. 1 includes the chip 10, an inlet 3 for supplying a fine particle-containing liquid to the chip 10, and an outlet 5 from which the fine particle-containing liquid having passed through the chip 10 is discharged. The configuration of the fine particle capture device of the invention is not limited to the one illustrated in FIG. 1 but, for instance, the whole of the fine particle capture device 1 of the invention shown in FIG. 1 may be covered with a casing.

As shown in FIG. 1 and FIG. 2, the chip 10 in the fine particle capture device 1 of the invention includes a flat part 12 and a large number of protruding parts 14 provided thereon.

As shown in FIG. 2, each of the protruding parts 14 preferably has a height (h) of 5 to 50 um and more preferably 8 to 20 µm.

In the fine particle capture device 1 of the invention as described above, the fine particle-containing liquid having entered through the inlet 3 flows toward the outlet 5 by the action of a pump, hydrostatic pressure, electroosmotic flow or the like. During this process, the fine particle-containing liquid flows on a surface of the flat part 12 in the chip 10, and through spaces each located between a protruding part 14 and another protruding part 14 adjacent thereto, and fine particles get caught and captured between specific protruding parts 14.

The fine particle-containing liquid is not particularly limited as long as it contains fine particles. Examples of the fine particle-containing liquid include human blood and a mixture liquid in which blood is dispersed in a buffer solution. The fine particle-containing liquid may also be a liquid in which fine particles with a particle size of about a few micrometers to a few hundred micrometers are dispersed. Specific examples thereof include a dispersion in which fine particulate matter such as PM10 and PM2.5 are dispersed, and a dispersion in which microplastic particles with an average particle size of about a few micrometers to a few hundred micrometers are dispersed.

The protruding parts 14 are provided on the flat part 12 in a layered form, as shown in FIG. 1.

FIG. 1 shows a layer closest to the inlet as a first layer, and a layer adjacent to the first layer on the outlet side (downstream side) as a second layer. FIG. 1 also shows a layer as a layer P, a layer adjacent to the layer P on the outlet side (downstream side) as a layer P+1, and a layer further adjacent thereto on the outlet side (downstream side) as a layer P+2.

Each layer contains a plurality of protruding parts 14. FIG. 1 shows an example in which each layer contains seven protruding parts 14. However, the number of the protruding parts 14 contained in each layer is not particularly limited. Further, the number of layers is also not particularly limited.

The fine particle-containing liquid having entered the fine particle capture device 1 of the invention through the inlet 3 flows over the surface of the flat part 12 to first pass through flow paths between the protruding parts 14 in the first layer and then pass through flow paths between the protruding parts 14 in the second layer. The fine particle capture device is configured so that the fine particle-containing liquid flows thereafter in the same manner to pass through flow paths between the protruding parts 14 in the layer P, and then pass through flow paths between the protruding parts 14 in the layer P+1.

The size and the material of the chip are not particularly limited. The chip may be made of, for example, resins such as silicone rubber, acrylic resin, polycarbonate, cyclic olefin polymer, cyclic olefin copolymer, polystyrene, polyethylene, and polyethylene terephthalate, and an embodiment in which resin is bonded to a substrate of glass or the like is preferable.

FIG. 3 is an enlarged view of the portion A in FIG. 1 in the case of the fine particle capture device of the invention.

As shown in FIG. 3, capture parts 21 and bypass parts 23 are formed in each layer of the fine particle capture device of the invention, each capture part 21 between a protruding part 14 and its adjacent protruding part 14 having a width (flow path width) L₁ set to be smaller than the diameter of fine particles to be captured, and each bypass part 23 having a width L₂ set to be larger than the diameter of fine particles to be captured.

The width L₃ between the layer P and the layer P+1 is preferably 7.5 to 30 µm, and more preferably 8 to 15 um.

The width L₃ means the shortest distance between the layer P and the layer P+1.

In the example of FIG. 3, in each layer of the layer P, the layer P+1, and the layer P+2, the capture parts 21 and the bypass parts 23 are alternately formed as flow paths between the plurality of protruding parts 14. In the fine particle capture device of the invention, however, the capture parts and the bypass parts formed in each layer may not be alternately formed as in FIG. 3. For instance, a plurality of capture parts may be successively present in each layer.

Further, a capture part 21 is disposed on an outlet side of a bypass part 23 in a specific layer as a part of another layer adjacent thereto. In other words, in the example of FIG. 3, a capture part 21 in the layer P+1 is disposed on the outlet side (downstream side) of a bypass part 23 in the layer P.

As illustrated in FIG. 3, a bypass part 23 in the layer P and a capture part 21 in the layer P+1 are preferably disposed side by side in a direction perpendicular to the layer direction. More specifically, the bypass part 23 in the layer P and the capture part 21 in the layer P+1 are preferably disposed so that, when a line in a direction perpendicular to the layer direction is drawn, the line passes through the bypass part 23 in the layer P and the capture part 21 in the layer P+1 (in other words, the line does not come into contact with the protruding parts 14).

In the example shown in FIG. 3, fine particles which are contained in the fine particle-containing liquid having flowed from the inlet side (upstream side) to reach the layer P are in principle not allowed to pass through the capture parts 21 and at least some of the fine particles are therefore captured in the capture parts 21. When the fine particles are captured, the capture parts 21 are closed. On the other hand, components other than the captured fine particles (including fine particles whose particle size is smaller) pass through the capture parts 21 in the layer P to reach the layer P+1. Further, all the components which are contained in the fine particle-containing liquid having reached the layer P are allowed to pass through the bypass parts 23. Therefore, fine particles which could not be captured in the capture parts 21 of the layer P pass through the bypass parts 23 in the layer P to reach the layer P+1, and at least some of them are captured in the capture parts 21 of the layer P+1. As the capture parts 21 in the layer P+1 are disposed on the outlet side (downstream side) of the bypass parts 23 in the layer P, the fine particles having passed through the bypass parts 23 in the layer P are easily captured in the capture parts 21 of the layer P+1.

The width of the capture parts can be modified for each layer.

For instance, when the fine particle-containing liquid contains fine particles with a relatively large particle size and fine particles with a relatively small particle size, and one wants to capture and separate them, the width of the capture parts in the chip is increased in the layers on the inlet side (upstream side) and is reduced in the layers on the outlet side (downstream side). In this case, by adjusting the width of the capture parts in the inlet side layers to be smaller than the diameter of the large size particles to be captured and larger than the diameter of the small size particles not to be captured, the large size fine particles can be singly captured in the capture parts in the inlet side layers. The small size particles pass through the capture parts in the inlet side layers but are captured in the capture parts on the outlet side by adjusting the width of the capture parts in the outlet side layers to be smaller than the diameter of the small size particles to be captured.

In the plan views as shown in FIG. 1 and FIG. 3 (that is, when the fine particle capture device 1 of the invention is seen from a direction perpendicular to the main surface of the chip 10), the protruding parts 14 preferably have a rectangular shape as shown in FIG. 1. Each protruding part 14 preferably has an approximately rectangular shape (a rectangular-based shape in which part of edges in four corners are linearly cut off to be chamfered, or a shape rounded by grinding at least part of edges in four corners of a rectangle) as shown in FIG. 3. The chamfered portion which is linearly cut off and the chamfered portion which is ground to have a round shape preferably each have an area which is half or less than half the area (projected area) of a fine particle to be captured.

In a case where the protruding parts 14 have a rectangular or approximately rectangular shape, other fine particles that reached the capture parts 21 already having fine particles captured therein move in the layer direction along the surfaces of the protruding parts 14 and move from the bypass parts 23 to the adjacent layer on the downstream side, where the fine particles are easily captured in the capture parts 21. Consequently, the inventor has found that the fine particle capture efficiency is increased. In a case where the protruding parts 14 do not have a rectangular shape (in the case of a circular shape or an elliptical shape, for example), their outer shape contains R and fine particles may therefore move along the R instead of moving to the capture parts 21 in the adjacent layer on the downstream side.

### EXAMPLES

### <Preparation of Fine Particle Capture Device>

A fine particle capture device was fabricated according to the procedure shown below. The width of capture parts in a chip was set to 15 um in layers on the inlet side (upstream side) and to 5 um in layers on the outlet side (downstream side). The width of bypass parts in the chip was set to 30 um in the upstream side layers and to 10 um in the layers on the outlet side (downstream side), and the width between specific layers and other layers adjacent thereto was set to 50 um without any exception.

First, a spinner was used to uniformly apply a photosensitive resin (SU-8 3050 manufactured by Nippon Kayaku Co., Ltd.) to a surface of a plate-like silicon wafer.

Next, the photosensitive resin was irradiated with ultraviolet light through a mask.

Next, the photosensitive resin on the silicon wafer exposed to the ultraviolet light was baked at 95°C.

Next, areas which were not exposed to the ultraviolet light were removed with a developer (SU-8 Developer manufactured by Nippon Kayaku Co., Ltd.) to fabricate a mold.

Next, silicone rubber (SILPOT184 manufactured by Dow Corning Corp.) was flowed into the mold.

Next, the silicone rubber was vulcanized under conditions of 100°C and 0.5 hours.

Next, the silicone rubber was peeled off from the silicon wafer to form a chip having flow paths formed therein.

Next, portions serving as an inlet and an outlet were perforated with punch holes to form a liquid introduction part, thereby fabricating the fine particle capture device.

### [Joining]

A light source (L 12530-01 manufactured by Hamamatsu Photonics K.K.) was used to irradiate both a glass substrate having the flow path-formed chip formed therein with vacuum ultraviolet light for 15 seconds. Then, both irradiated surfaces were bonded together to form a chip.

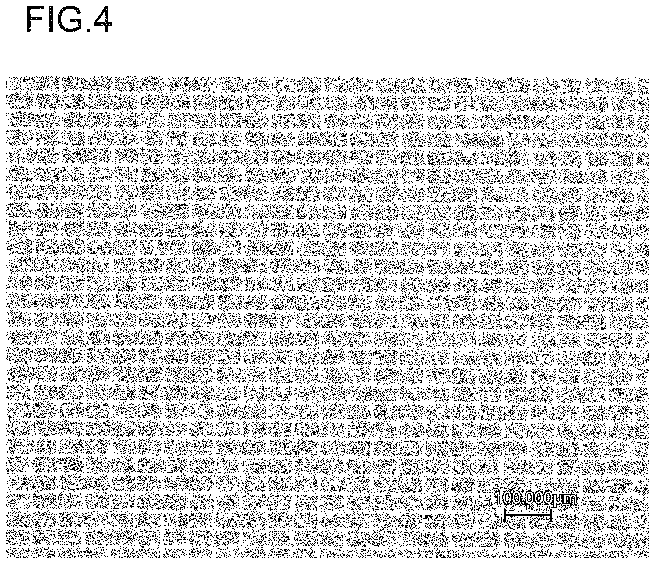

The formed chip was observed with a stereoscopic microscope and an enlarged photo obtained is shown in FIG. 4.

### <Experiments>

Three types of polystyrene beads having diameters of 25 um, 6 µm, and 1 µm, respectively were dispersed in a phosphate buffer solution (PBS(-) manufactured by Wako Pure Chemical Industries, Ltd.) to adjust a reagent.

Next, a pipette was used to flow the reagent in the fine particle capture device.

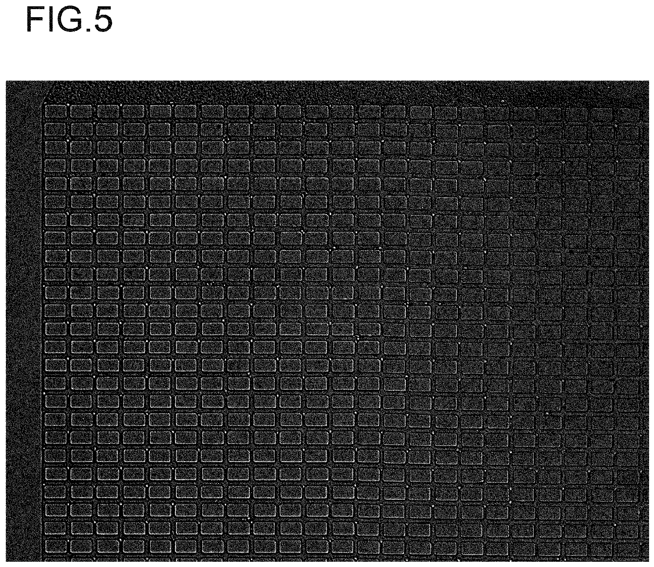

Then, the chip of the fine particle capture device was observed with the stereoscopic microscope. An enlarged photo obtained by observing with the stereoscopic microscope is shown in FIG. 5. The magnification in FIG. 5 is the same as that in FIG. 4.

The enlarged photo revealed that the polystyrene beads with the diameter of 25 um and the polystyrene beads with the diameter of 6 um could be singly captured in the capture parts in the upstream side layers and in the capture parts in the downstream side layers, respectively.

This application claims priority based on Japanese Patent Application No. 2020-163376 filed on September 29, 2020, the entire disclosure of which is incorporated herein by reference.

### REFERENCE SIGNS LIST

- 1: fine particle capture device of the invention
- 3: inlet
- 5: outlet
- 10: chip
- 12: flat part
- 14: protruding part
- 21: capture part
- 23: bypass part

## Claims

1. A fine particle capture device comprising: a chip for passing a fine particle-containing liquid therethrough and capturing fine particles contained in the fine particle-containing liquid,
wherein the chip has a flat part and a large number of protruding parts provided thereon, and is configured so that the fine particle-containing liquid having entered through an inlet passes on a surface of the flat part in the chip, and through spaces each located between a protruding part and another protruding part adjacent thereto and is discharged from an outlet,
wherein the protruding parts are provided on the flat part in a layered form, each layer has a plurality of protruding parts, and the protruding parts are configured so that the fine particle-containing liquid having passed through a layer on an inlet side passes through a layer adjacent thereto on an outlet side,
wherein capture parts and bypass parts are formed in each layer, a width between a protruding part and its adjacent protruding part being set to be smaller in the capture parts and larger in the bypass parts than a diameter of the fine particles to be captured, and
wherein capture parts are disposed on an outlet side of bypass parts in a specific layer as a part of another layer adjacent thereto.

2. The fine particle capture device according to claim 1, wherein the protruding parts have a rectangular or approximately rectangular shape when seen from a direction perpendicular to a main surface of the chip.
